# EUROPEAN PATENT SPECIFICATION

(11) **EP 2 398 546 B1**
(45) Date of publication and mention of the grant of the patent: **18.11.2020**
(21) Application number: 10704879.5
(22) Date of filing: 17.02.2010
(51) Int. Cl.: A61M 25/10, A61M 25/00, A61M 25/01

(54) **BALLOON CATHETER**
BALLONKATHETER
CATHÉTER À BALLONNET

(30) Priority: 20.02.2009 US 390153
(43) Date of publication of application: 28.12.2011
(73) Proprietor: Boston Scientific Scimed, Inc., Maple Grove, MN 55311 (US)
(72) Inventor: Gregorich, Daniel, St. Louis Park Minnesota 55416 (US); Rowe, Todd, Chanhassen Minnesota 55317 (US); Rundquist, Chuck, White Bear Lake Minnesota 55110 (US); Gundale, Ben, Plymouth Minnesota 55447 (US)
(74) Representative: Vossius & Partner Patentanwälte Rechtsanwälte mbB
(86) International application number: PCT/US2010/024465
(87) International publication number: WO 2010/096476

(56) References cited:
- EP-A1- 0 998 955
- EP-A2- 0 430 542
- WO-A2-97/48326
- WO-A2-2008/033998
- US-A- 5 344 402
- US-A- 6 059 770
- US-B1- 7 195 611

## Description

### Technical Field

The present invention relates generally to medical devices and more particularly to balloon catheters.

### Background

Heart and vascular disease are major problems in the United States and throughout the world. Conditions such as atherosclerosis result in blood vessels becoming blocked or narrowed. This blockage can result in lack of oxygenation of the heart, which has significant consequences since the heart muscle must be well oxygenated in order to maintain its blood pumping action.

Occluded, stenotic, or narrowed blood vessels may be treated with a number of relatively non-invasive medical procedures including percutaneous transluminal angioplasty (PTA), percutaneous transluminal coronary angioplasty (PTCA), and atherectomy. Angioplasty techniques typically involve the use of a balloon catheter. The balloon catheter is advanced over a guidewire so that the balloon is positioned adjacent a stenotic lesion. The balloon is then inflated, and the restriction of the vessel is opened.

There is an ongoing need for improved angioplasty devices, including balloon catheters.

EP 0 998 955 discloses a balloon catheter for angioplasty or for placing a reinforcing stent into a human vessel which has a variable stiffness catheter portion for guiding the catheter through the vasculature. The catheter portion of the balloon catheter includes a relatively stiff shaft portion which is reinforced with braided layer and more flexible distal portion which is reinforced with a single helically wire coil.

### Summary

The invention pertains to improved medical devices providing advantages in flexibility, strength and other desired properties.

The balloon catheter of the present invention includes a coil assembly defining a guidewire lumen. The coil assembly includes a coil, a coating disposed on the coil, and a sheath disposed over the coil and the coating. An outer tube disposed over at least a portion of the coil assembly. A balloon may be coupled to the outer tube wherein an inflation lumen is defined between the coil assembly and the outer tube. A balloon coupled to the outer tube; and a tip; wherein the coil assembly extends distally beyond the end of the balloon and distally along a portion of the tip.

An example coil assembly for use in a medical device may include a ribbon coil. A fluoropolymer coating may be disposed on the ribbon coil. The coating may be disposed along an interior surface of the ribbon coil and along an exterior surface of the ribbon coil. A sheath may be disposed about the ribbon coil and the coating.

An example method of forming a coil assembly may include providing a flat ribbon coil member and coating the flat ribbon coil member with a fluoropolymer. After the coating step, the method may include coiling the flat ribbon coil member to form a coated coil and disposing a sheath about an exterior of the coated coil. Adjacent windings of the coated coil may be in contact with one another.

The above summary of the present invention is not intended to describe each disclosed embodiment or every implementation of the present invention. The Figures, Detailed Description and Examples which follow more particularly exemplify these embodiments.

### Brief Description of the Figures

The invention may be more completely understood in consideration of the following detailed description of various embodiments of the invention in connection with the accompanying drawings, in which:
Figure 1 is a schematic view of an illustrative but non-limiting balloon catheter in accordance with the present disclosure;
Figure 2 is a schematic partial cross-sectional view of a portion of an illustrative but non-limiting balloon catheter in accordance with the present disclosure;
Figure 3 is a schematic partial cross-sectional view of a portion of an illustrative but non-limiting balloon catheter in accordance with the present disclosure;
Figure 4 is a schematic partial cross-sectional view of a portion of an illustrative but non-limiting balloon catheter in accordance with the present disclosure;
Figure 5 is a cross-sectional view of an example coil assembly;
Figure 6 is a cross-sectional view of a distal portion of a balloon catheter according to the present invention.; and
Figure 7 is a cross-section view of a guidewire port of the balloon catheter illustrated in Figure 6.

While the invention is amenable to various modifications and alternative forms, specifics thereof have been shown by way of example in the drawings and will be described in detail. It should be understood, however, that the intention is not to limit the invention to the particular embodiments described. On the contrary, the intention is to cover all modifications, equivalents, and alternatives falling within the scope of the invention.

### Detailed Description

For the following defined terms, these definitions shall be applied, unless a different definition is given in the claims or elsewhere in this specification.

All numeric values are herein assumed to be modified by the term "about", whether or not explicitly indicated. The term "about" generally refers to a range of numbers that one of skill in the art would consider equivalent to the recited value (i.e., having the same function or result). In many instances, the terms "about" may include numbers that are rounded to the nearest significant figure.

The recitation of numerical ranges by endpoints includes all numbers within that range (e.g., 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

The following description should be read with reference to the drawings wherein like reference numerals indicate like elements throughout the several views. The drawings, which are not necessarily to scale, depict illustrative embodiments of the claimed invention.

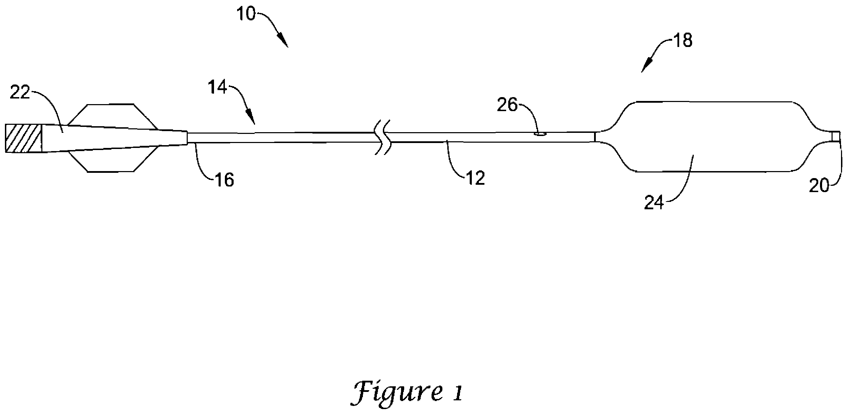

Figure 1 is a plan view of a catheter 10 in accordance with an example of the present disclosure.

The catheter 10 can be any of a variety of different catheters. For example, the catheter 10 can be an intravascular catheter. Examples of intravascular catheters include balloon catheters, atherectomy catheters, drug delivery catheters, stent delivery catheters, diagnostic catheters and guide catheters. The intravascular catheter 10 can be sized in accordance with its intended use. The catheter 10 can have a length that is in the range of about 100 to 150 centimeters and can have any useful diameter. As illustrated, Figure 1 portrays a balloon catheter, but the disclosure is not limited to such. Except as described herein, the intravascular catheter 10 can be manufactured using conventional techniques.

In the illustrated example, the intravascular catheter 10 includes an elongate shaft 12 that has a proximal region 14 defining a proximal end 16 and a distal region 18 defining a distal end 20. A hub and strain relief assembly 22 can be connected to the proximal end 16 of the elongate shaft 12. The hub and strain relief assembly 22 can be of conventional design and can be attached using conventional techniques. It is also recognized that alternative hub designs can be incorporated into embodiments of the present invention.

The elongate shaft 12 can include one or more shaft segments having varying degrees of flexibility. For example, the elongate shaft may include a relatively stiff proximal portion, a relatively flexible distal portion and an intermediate position disposed between the proximal and distal portions having a flexibility that is intermediate to both.

In some cases, the elongate shaft 12 or portions thereof may be formed of a single polymeric layer. In some instances, the elongate shaft 12 may include an inner liner such as an inner lubricious layer and an outer layer. In some cases, the elongate shaft 12 may include a reinforcing braid layer disposed between the inner and outer layers. The elongate shaft 12 is considered herein as generically representing a catheter to which various elements can be added to provide the catheter 10 with adjustable stiffness.

If the elongate shaft 12 includes an inner liner, the inner liner can include or be formed from a coating of a material having a suitably low coefficient of friction. Examples of suitable materials include perfluoro polymers such as polytetrafluoroethylene (PTFE), better known as TEFLON®, high density polyethylene (HDPE), polyarylene oxides, polyvinylpyrolidones, polyvinylalcohols, hydroxy alkyl cellulosics, algins, saccharides, caprolactones, and the like, and mixtures and combinations thereof.

The elongate shaft 12 can include, as an outer layer or layers, any suitable polymer that will provide the desired strength, flexibility or other desired characteristics. Polymers with low durometer or hardness can provide increased flexibility, while polymers with high durometer or hardness can provide increased stiffness. In some embodiments, the polymer material used is a thermoplastic polymer material. Some examples of suitable materials include polyurethane, elastomeric polyamides, block polyamide/ethers (such as PEBAX®), silicones, and co-polymers. The outer polymer layer can be a single polymer, multiple longitudinal sections or layers, or a blend of polymers. In some instances, a thermoplastic polymer such as a co-polyester thermoplastic elastomer, for example, available commercially under the ARNITEL® name, can be used.

In some instances, elongate shaft 12 or portions thereof may include or be formed from one or more metallic materials. In some cases, metals may be used in combination with one or more polymers such as those discussed above. Examples of suitable metals for inclusion in part or all of elongate shaft 12 include stainless steel, such as 300 series stainless steel (including 304V, 304L, and 316L; 400 series martensitic stainless steel; tool steel; nickel-titanium alloy such as linear-elastic or super-elastic Nitinol, nickel-chromium alloy, nickel-chromium-iron alloy, cobalt alloy, tungsten or tungsten alloys, MP35-N (having a composition of about 35% Ni, 35% Co, 20% Cr, 9.75% Mo, a maximum 1% Fe, a maximum 1% Ti, a maximum 0.25% C, a maximum 0.15% Mn, and a maximum 0.15% Si), hastelloy, monel 400, inconel 625, or the like; or other suitable material.

The catheter 10 also includes an inflatable balloon 24 that is disposed about the elongate shaft 12 within the distal region 18 thereof. The balloon 24 may be made from typical angioplasty balloon materials including polymers such as polyethylene terephthalate (PET), polyetherimide (PEI), polyethylene (PE), etc. Some other examples of suitable polymers, including lubricious polymers, may include polytetrafluoroethylene (PTFE), ethylene tetrafluoroethylene (ETFE), fluorinated ethylene propylene (FEP), polyoxymethylene (POM), polybutylene terephthalate (PBT), polyether block ester, polyurethane, polypropylene (PP), polyvinylchloride (PVC), polyether-ester (for example, and a polyether-ester elastomer such as ARNITEL® available from DSM Engineering Plastics).

Additional examples of suitable polymers include polyester (for example, a polyester elastomer such as HYTREL® available from DuPont), polyamide (for example, DURETHAN® available from Bayer or CRISTAMID® available from Elf Atochem), elastomeric polyamides, block polyamide/ethers, nylons such as polyether block amide (PEBA, for example, available under the trade name PEBAX®), silicones, Marlex high-density polyethylene, Marlex low-density polyethylene, linear low density polyethylene (for example, REXELL®), polyetheretherketone (PEEK), polyimide (PI), polyphenylene sulfide (PPS), polyphenylene oxide (PPO), polysulfone, nylon, perfluoro(propyl vinyl ether) (PFA), other suitable materials, or mixtures, combinations, copolymers thereof, polymer/metal composites, and the like.

In some cases, it may be desirable to use high modulus or generally stiffer materials so as to reduce balloon elongation. The above list of materials includes some examples of higher modulus materials. Some other examples of stiffer materials include polymers blended with liquid crystal polymer (LCP) as well as the materials listed above. For example, the mixture can contain up to about 5% LCP.

In some cases, the catheter 10 may represent an over-the-wire (OTW) catheter in which a guidewire (not illustrated) may, in use, enter the catheter 10 via proximal hub 22 and may exit through an opening at the distal end 20. In some instances, the catheter 10 may represent a rapid-exchange, or single-operator-exchange catheter (SOE) in which a guidewire may pass through a shorter guidewire lumen extending proximally from the distal end 20 to a guidewire port 26. In some cases, it will be recognized that the catheter 10 may be adapted to accommodate both OTW and SOE operation.

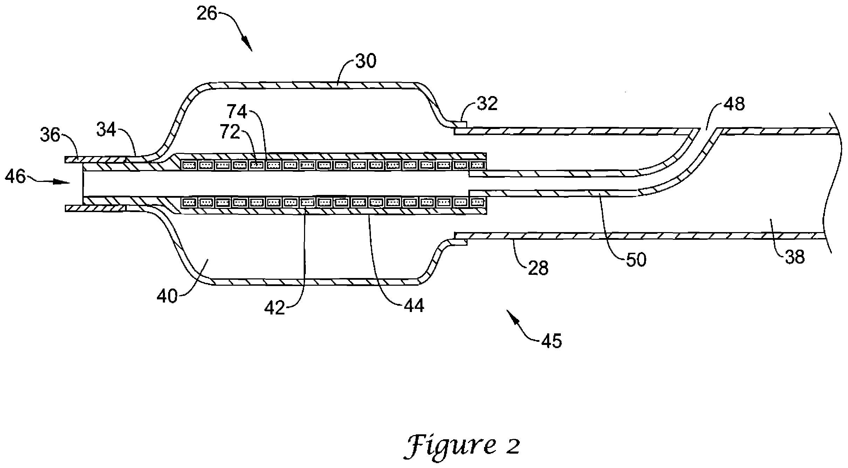

Figure 1 provides an overview of the catheter 10. Figures 2 through 5 provide greater detail regarding features of the catheter 10, particularly within the distal region 18 thereof. Figure 2 is a schematic cross-sectional view of a distal portion of a catheter 26 that, aside from features specifically discussed with respect to Figure 2, shares many features in common with the catheter 10 discussed with respect to Figure 1.

The catheter 26 includes an elongate shaft 28 that may be constructed in accordance with the materials discussed previously with respect to elongate shaft 12 (Figure 1). A balloon 30 having a proximal waist 32 and a distal waist 34 is disposed about the elongate shaft 28, and may be secured to the elongate shaft 28 using any suitable technique such as laser welding, thermal bonding, adhesive and the like. The balloon 30 may be formed of any suitable polymeric materials, such as those described with respect to Figure 1. In some cases, the balloon 30 may include or be formed from a polyamide such as PEBAX® 7233.

If desired, a bumper tip 36 may be secured distal of the balloon 30, but this is not required. If present, the bumper tip 36 may be formed of any suitably soft polymeric material to reduce potential tissue damage.

The elongate shaft 28 includes an inflation lumen 38 that is in fluid communication with an interior 40 of the balloon 30 and that can be used to provide sufficient inflation fluid (saline or the like) to inflate or deflate the balloon 30 as desired. The elongate shaft 28 may be formed of any suitable metallic or polymeric material such as those discussed with respect to Figure 1. In some instances, the elongate shaft 28 may include or be formed from a polyamide such as PEBAX®. In particular instances, the elongate shaft 28 may include or be formed from PEBAX® 7033.

Disposed within the interior 40 of the balloon 30 is a coil 42 that is covered by a polymer sheath 44. In many cases, the polymer sheath 44 is a polyamide heat shrink tube that has been, via application of heat and/or pressure, shrunk down onto the coil 42. The polymer sheath 44 may, in some instances, include or be formed from a polyamide such as PEBAX® having a durometer ranging from about 40D to about 70D. In particular cases, the polymer sheath 44 may be formed of a PEBAX® having a durometer of about 63D.

In some instances, as illustrated, the polymer sheath 44 may extend all the way to a distal end 46 of the catheter 26. It will be recognized that in forming the catheter 26, a mandrel (not illustrated) may be temporarily inserted into the distal end of the polymer sheath 44 prior to shrinking the polymer to retain a guidewire lumen therethrough.

In some cases, the polymer sheath 44 may not extend all the way to the distal end 46, but may extend at least to a position proximate the distal waist 34. As a result, the combination of the coil 42 and the polymer sheath 44 may provide a fluid-tight passage through the balloon 30 such that a guidewire (not illustrated) may pass through while not interfering with an ability to inflate and/or deflate the balloon 30 as desired.

In some instances, the coil 42 may be formed from a coil wire that has been polymer coated before the coil wire is wrapped or coiled into a coil form. In some cases, the coil wire is coated with a fluoropolymer such as polytetrafluoroethylene prior to being coiled into the coil 42. This can result in a coil 42 that has a fluoropolymer coating on an interior of the coil 42. This provides reduced friction for any guidewire advanced through the coil 42.

In some cases, it is contemplated that other techniques may be used to provide a fluoropolymer coating on an interior of the coil 42. In some instances, the fluoropolymer coating may be applied via sputter coating or dip coating, for example.

Any suitable coil wire may be used to form the coil 42. In some cases, the coil 42 may be formed from a flat ribbon coil wire having a relatively flat rectangular profile. In some instances, the flat ribbon coil wire may have a width-to-height ratio of about 3:1, about 4:1, about 5:1, about 6:1, about 7:1 or even wider. In particular cases, the flat ribbon coil wire may, for example, have a cross-sectional width of about seven thousands of an inch and a height of about 1 thousands of an inch. The coil wire used to form the coil 42 may be formed of any polymeric or metallic material, such as those materials recited above. In particular instances, the coil 42 may be formed from a stainless steel coil wire. It can be seen that each individual coil turning 72 is at least substantially if not completely coated with a polymeric coating 74.

As illustrated, the catheter 26 is adapted to provide SOE functionality. The catheter 26 includes a guidewire port 48 that leads to a guidewire lumen 50. The guidewire port 48 is disposed within a distal region 45 of the catheter 26 but is proximal of the balloon 30. In some instances, the polymer sheath 44 covering the coil 42 extends proximally a sufficient distance to form a fluid-tight seal with the guidewire lumen 50. As a result, the guidewire port 48 does not interfere with an ability to inflate and/or deflate the balloon 30 as desired. In some cases, the polymer sheath 44 is heat-shrunk onto the guidewire lumen 50. In some instances, the guidewire lumen 50 may instead be adhesively secured to the coil 42 and/or the polymer sheath 44.

The guidewire lumen 50 may be formed in any suitable manner and of any suitable material. In some cases, the guidewire lumen 50 may be formed by extending a polymeric tube between the coil 42 and the guidewire port 48. In some instances, the guidewire lumen 50 may be a metallic construct. In many instances, the construction shown within the distal portion of the catheter 26 may provide for improved pushability and improved flexibility all the way to the distal end 46.

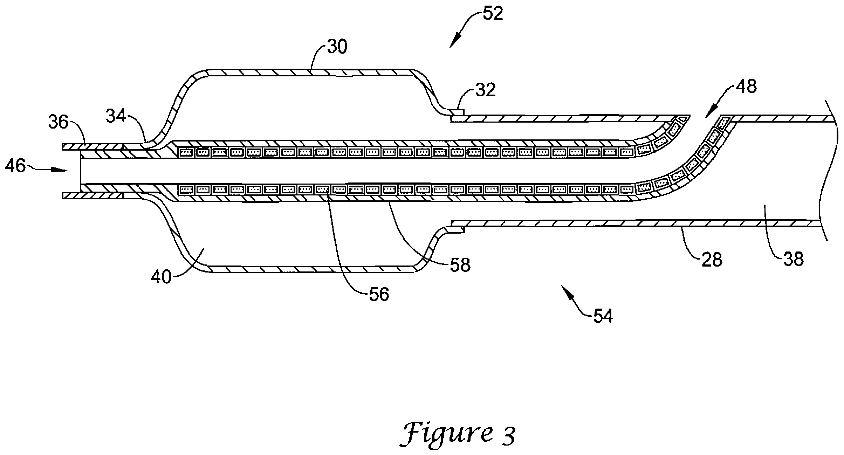

Figure 3 is a schematic cross-sectional view of a distal portion of a catheter 52 that, aside from features specifically discussed with respect to Figure 3, shares many features in common with the catheter 10 discussed with respect to Figure 1 as well as the catheter 26 discussed with respect to Figure 2.

The catheter 52 is also an SOE catheter, having a guidewire port 48 that is disposed within a distal region 54 of the catheter 52 but is proximal of the balloon 30. The catheter 52 includes a coil 56 that extends from a position within the balloon 30 at or near the distal waist 34 all the way to the guidewire port 48. A polymer sheath 58 extends at least from a position at or near the distal waist 34 (if it does not extend all the way to the distal end 46) to the guidewire port 48.

The coil 56 and the polymer sheath 58 are formed in a manner analogous to that described with respect to the coil 42 and the polymer sheath 44, respectively, of Figure 2. In some instances, the coil 56 is formed of a flat ribbon coil wire such as stainless steel that has been coated with a fluoropolymer such as polytetrafluoroethylene prior to coiling. In some cases, the polymer sheath 58 may, in some instances, include or be formed from a polyamide such as PEBAX® having a durometer ranging from about 40D to about 70D.

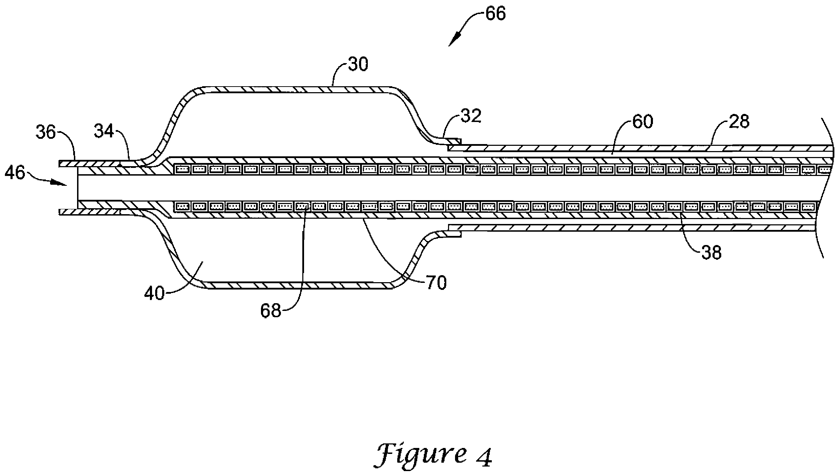

Figure 4 illustrates an OTW catheter 66 having a coil 68 and a polymer sheath 70. As illustrated, the coil 68 and the polymer sheath 70 extend proximally an indefinite distance. In some cases, the coil 68 and the polymer sheath 70 may extend proximally only about as far as the balloon 30 extends. In some instances, the coil 68 and the polymer sheath 70 may extend all the way to a proximal hub (not illustrated), or may terminate at some intermediate position. An annular inflation lumen 60 extends between the elongate shaft 28 and the polymer sheath 70.

The coil 68 is constructed in accordance with the materials and techniques discussed with respect to the coil 42. Similarly, the polymer sheath 70 is constructed in accordance with the materials and techniques discussed with respect to the polymer sheath 44. In some instances, the coil 68 is formed of a flat ribbon coil wire such as stainless steel that has been coated with a fluoropolymer such as polytetrafluoroethylene prior to coiling. In some cases, the polymer sheath 70 may, in some instances, include or be formed from a polyamide such as PEBAX® having a durometer ranging from about 40D to about 70D.

In some examples, part or all of the devices described herein can include a lubricious coating. Lubricious coatings can improve steerability and improve lesion crossing capability. Examples of suitable lubricious polymers include hydrophilic polymers such as polyarylene oxides, polyvinylpyrolidones, polyvinylalcohols, hydroxy alkyl cellulosics, algins, saccharides, caprolactones, and the like, and mixtures and combinations thereof. Hydrophilic polymers can be blended among themselves or with formulated amounts of water insoluble compounds (including some polymers) to yield coatings with suitable lubricity, bonding, and solubility. In some examples, portions of the devices described herein can be coated with a hydrophilic polymer or a fluoropolymer such as polytetrafluoroethylene (PTFE), better known as TEFLON®.

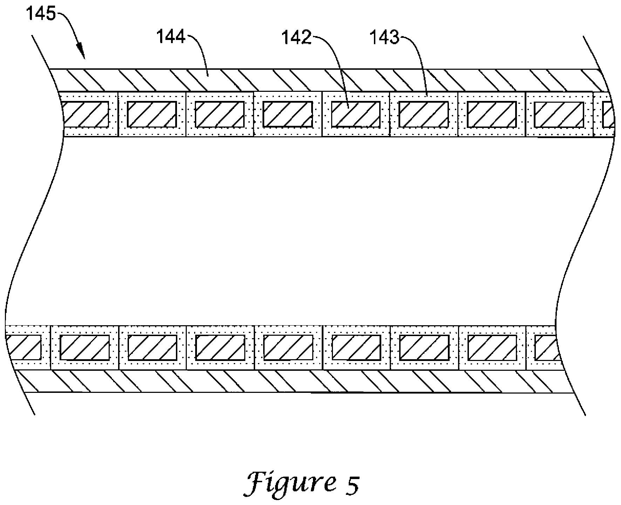

Figure 5 illustrates a portion of an example coil assembly 145 that may be similar in form and function to coil 42 and/or the various components associated therewith. To the extent applicable, any discussion above related to coil 42 and/or the various components associated therewith may be applied to coil assembly 145 (and/or the components thereof) and vice versa. Coil assembly 145 may be used as or otherwise define a tube or tubular member that may form a medical device and/or a component of a medical device. For example, coil assembly 145 may be used as a tubular member that makes up a catheter shaft or a portion of a catheter shaft. Alternatively, coil assembly 145 may be used to provide structural support to a catheter shaft or a portion of a catheter shaft. Numerous other examples are also contemplated.

Coil assembly 145 may include a coil 142, a coating 143 that covers coil 142, and a sheath 144 that covers the coated coil 142 (e.g., coil 142 and coating 143 disposed thereon). Coil 142 may include a metal such as any of the metal disclosed herein. For example, coil 142 may include stainless steel. Coating 143 may be a polymer coating that may include any of the polymers disclosed herein. For example, coating 143 may include a fluoropolymer such as polytetrafluoroethylene. Sheath 144 may likewise include a polymer such as any of the polymers disclosed herein. For example, sheath 144 may include a polyamide such as PEBAX®.

Coil 142 may have a configuration and dimensions suitable for its intended use. For example, coil 142 may take the form of a ribbon coil (as shown). Other coil configurations are also contemplated including round coils or coils having different shapes or configurations. The ribbon width of coil 142 may be in the range of about 0,0254 to about 1,27 mm (0,001 to about 0,05 inches) or in the range of about 0,127 to 0,508 mm (0,005 to 0,02 inches), or about 0,254 mm (0,01 inches). The ribbon height or thickness may be in the range of about 0,00254 to about 0,127 mm (0,0001 to about 0,005 inches) or in the range of about 0,0127 to about 0,0508 mm (about 0,0005 to about 0,002 inches), or about 0,0254 mm (0,001 inches). A suitable thickness of coating 143 may be applied to coil 142. For example, coating 143 may have a thickness in the range of about 0,00254 to about 0,0254 mm (about 0,0001 to about 0,001 inches), or in the range of about 0,00254 to about 0,0127 mm (bout 0,0001 to about 0,0005 inches), or about 0,00508 mm (0,0002 inches).

In some examples, coating 143 may be applied to coil 142 prior to winding coil 142. This may be desirable for a number of reasons including some of those listed herein. For example, applying coating 143 to coil 142 prior to winding may allow coating 143 to be disposed along both the interior and the exterior of the wound coil 142 (as well as along the sides or side surfaces of coil 142). The coated coil 142 may be wound "tight" (e.g., essentially with no gaps between adjacent windings of the coated coil 142) and to the appropriate dimensions. This may be understood to mean that the either the adjacent windings of coil 142 directly contact one another (e.g., in examples where the side surfaces of coil 142 do not bear coating 143) or that coating 143 on adjacent windings of the coated coil 142 directly contact one another (e.g., in examples where the side surfaces of coil 142 bear coating 143). For example, coil 142 may be wound to 0,4191 mm (0,0165 inches) 0,127 mm (0,005 inches) inner diameter by 0,5461 mm (0,0215 inches) +/- 0,127 mm (0,005 inches) outer diameter. In order to minimize the likelihood of gaps forming between windings of coil 142, coil 142 may be wound with pre-tension (e.g., such that if coil 142 is stretched longitudinal, causing gaps to form, coil 142 with "snap back" to a tight configuration). Pre-tension can be measured using a droop test with suitable droop value ranges of about 24,4 to 127 mm (1 to 5 inches) or about 38,1 to 88,9 mm (1,5 to 3,5 inches) when measured with a 19,05 mm (0,75 inch) gauge block.

By virtue of winding coil 142 in a tight configuration, coil assembly 145 may have increased push performance. For example, because adjacent windings of coil 142 may be disposed directly against one another, push forces (e.g., compression) may be transferred directly from one winding to another, thereby resulting in push forces at one end of coil assembly 145 transferring more accurately to the opposite end of coil assembly 145. Consequently, devices that include coil assembly 145 may have enhanced pushability and/or push performance.

Sheath 144 may be disposed on the coated coil 142 in any suitable manner. For example, sheath 144 may be formed of a heat shrink tube that is disposed over coil 142 and subsequently shrunk onto the coated coil 142. Forming the heat shrink tube may include extruding a polyamide (e.g., PEBAX®) tube (e.g., having an inner diameter of about 0,3048 to 0,4064 mm (0,012 to 0,016 inches) and a wall thickness of about 0,0254 +/- 0,0127 mm (0.001 +/-0.0005 inches) when disposed over coil 142). The heat shrink tube may be expanded (e.g., so as to have an inner diameter of about 0,635 +/- 0,254 mm ( 0.025 +/- 0.01 inches)) to facilitate placement over coil 142. Once placed over coil 142, the tube can be heated with an appropriate heat source such as hot air to shrink the tube onto the coated coil 142 and form coil assembly 145. The assembled coil assembly 145 may be incorporated into a number of different medical devices as described herein.

As indicated above, the materials for the tube that forms sheath 144 may include polyamide. However, any suitable material may be utilized including those polymers disclosed herein. In some embodiments, the material selected for sheath 144 may have a durometer of about 40D to about 80D, or about 55D to about 72D, or about 63D.

Coil assembly 145 may be incorporated into a number of different medical devices. For example, as indicated above coil assembly 145 may form or otherwise be a component of essentially any suitable medical device such as a guiding, diagnosing, or treating device (including guidewires, catheters, guide catheters, balloon catheters, stent delivery devices, endoscopic instruments, laparoscopic instruments, etc., and the like) and it may be suitable for use at essentially any location and/or body lumen within a patient.

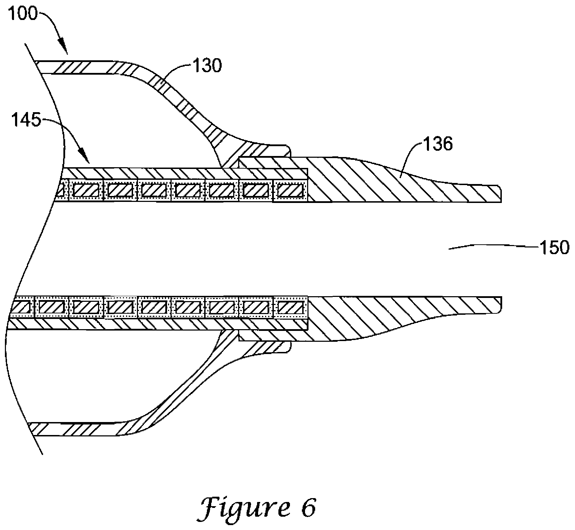

Figure 6 illustrates medical device 100 including coil assembly 145. Medical device 100 may be similar in form and function to other devices (e.g., catheters and/or balloon catheters such as catheter 10) disclosed herein and takes the form of a balloon catheter. To the extent applicable, any discussion above that is related to similar devices (including catheter 10) and/or components thereof may be applied to catheter 100 (and/or components thereof). Catheter 100 includes a balloon 130 and a tip 136 coupled to coil assembly 145 and balloon 130. Coil assembly 145 takes the form of an inner shaft of catheter 100 and defines a guidewire lumen 150 therethrough.

In the present invention, coil assembly 145 : extends distally beyond the end of balloon 30 and distally along a portion of tip 136. In some of these as well as other embodiments, coil assembly 145 may extend distally up to a position adjacent the end of tip 136, about at the end of tip 136, or to the end of tip 136.

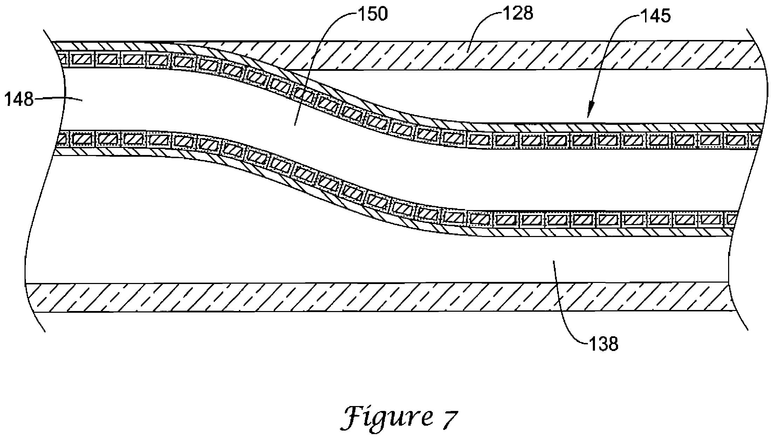

Figure 7 shows a more proximal portion of catheter 100 and illustrates that catheter 100 also includes an outer tube 128. Outer tube 128 is disposed over at least a portion of coil assembly 145. An inflation lumen 138 is formed between coil assembly 145 and outer tube 128, which is used to inflate balloon 130. In some embodiments, catheter 100 may be a SOE catheter. Accordingly, coil assembly 145 may extend proximally and form a proximal guidewire port 128 on catheter 100. Other embodiments are contemplated where catheter 100 is an OTW catheter or other suitable device.

The invention should not be considered limited to the particular examples described above, but rather should be understood to cover all aspects of the invention as set out in the attached claims. Various modifications, equivalent processes, as well as numerous structures to which the invention can be applicable will be readily apparent to those of skill in the art upon review of the instant specification.

## Claims

1. A balloon catheter (100), comprising:
a coil assembly (145), the coil assembly including a coil (142), a coating (143) disposed on the coil, wherein each individual coil turning is at least substantially coated, and a sheath (144) disposed over the coil (142) and the coating (143), the coil assembly (145) defining a guidewire lumen (150);
an outer tube (128) disposed over at least a portion of the coil assembly (145), wherein an inflation lumen (138) is defined between the coil assembly (145) and the outer tube (128);
a balloon (130) coupled to the outer tube (128); and
a tip (136);
**characterised in that**
the coil assembly (145) extends distally beyond the end of the balloon (130) and distally along a portion of the tip (136).

2. The balloon catheter (100) of claim 1, wherein the coil (142) includes a ribbon coil.

3. The balloon catheter (100) of claim 1 or 2, wherein the coating (143) is disposed along an interior surface of the coil (142).

4. The balloon catheter (100) of claim 3, wherein the coating (143) is disposed along an exterior surface of the coil (142).

5. The balloon catheter (100) of any of claims 1 to 4, wherein the coating (143) includes a fluoropolymer.

6. The balloon catheter (100) of any of claims 1 to 5, wherein the coil (142) includes stainless steel.

7. The balloon catheter (100) of any of claims 1 to 6, wherein the coating (143) includes polytetrafluoroethylene.

8. The balloon catheter (100) of any of claims 1 to 7, wherein the sheath (144) includes a polyamide.

9. The balloon catheter (100) of any of claims 1 to 8, wherein the sheath (144) includes a heat shrink tube.

10. The balloon catheter (100) of any of claims 1 to 9, wherein the coil assembly (145) extends distally up to a position adjacent the end of the tip (136), or to the end of the tip (136).

11. A medical device including a balloon catheter (100) according to any preceding claim.

## Patentansprüche

1. Ballonkatheter (100), der aufweist:
eine Spiralanordnung (145), wobei die Spiralanordnung aufweist: eine Spirale (142), eine Beschichtung (143), die auf der Spirale angeordnet ist, wobei jede einzelne Spiralwindung mindestens im Wesentlichen beschichtet ist, und eine Hülle (144), die über der Spirale (142) und der Beschichtung (143) angeordnet ist, wobei die Spiralanordnung (145) ein Führungsdrahtlumen (150) bildet;
eine Außenröhre (128), die über mindestens einem Abschnitt der Spiralanordnung (145) angeordnet ist, wobei ein Inflationslumen (138) zwischen der Spiralanordnung (145) und der Außenröhre (128) gebildet ist;
einen Ballon (130), der mit der Außenröhre (128) gekoppelt ist; und
eine Spitze (136);
**dadurch gekennzeichnet, dass**
sich die Spiralanordnung (145) distal über das Ende des Ballons (130) hinaus und
distal entlang eines Abschnitts der Spitze (136) erstreckt.

2. Ballonkatheter (100) nach Anspruch 1, wobei die Spirale (142) eine Bandspirale aufweist.

3. Ballonkatheter (100) nach Anspruch 1 oder 2, wobei die Beschichtung (143) entlang einer Innenfläche der Spirale (142) angeordnet ist.

4. Ballonkatheter (100) nach Anspruch 3, wobei die Beschichtung (143) entlang einer Außenfläche der Spirale (142) angeordnet ist.

5. Ballonkatheter (100) nach einem der Ansprüche 1 bis 4, wobei die Beschichtung (143) ein Fluorpolymer aufweist.

6. Ballonkatheter (100) nach einem der Ansprüche 1 bis 5, wobei die Spirale (142) Edelstahl aufweist.

7. Ballonkatheter (100) nach einem der Ansprüche 1 bis 6, wobei die Beschichtung (143) Polytetrafluorethylen aufweist.

8. Ballonkatheter (100) nach einem der Ansprüche 1 bis 7, wobei die Hülle (144) ein Polyamid aufweist.

9. Ballonkatheter (100) nach einem der Ansprüche 1 bis 8, wobei die Hülle (144) einen Warmschrumpfschlauch aufweist.

10. Ballonkatheter (100) nach einem der Ansprüche 1 bis 9, wobei sich die Spiralanordnung (145) distal bis zu einer Position benachbart zum Ende der Spitze (136) oder bis zum Ende der Spitze (136) erstreckt.

11. Medizinprodukt mit einem Ballonkatheter (100) nach einem der vorstehenden Ansprüche.

## Revendications

1. Cathéter à ballonnet (100), comprenant :
un ensemble à bobine (145), l'ensemble à bobine incluant une bobine (142), un revêtement (143) disposé sur la bobine, dans lequel chaque tour de bobine individuel est au moins sensiblement revêtu, et une gaine (144) disposée sur la bobine (142) et le revêtement (143), l'ensemble à bobine (145) définissant une lumière pour fil de guidage (150) ;
un tube externe (128) disposé sur au moins une partie de l'ensemble à bobine (145), dans lequel une lumière de gonflage (138) est définie entre l'ensemble à bobine (145) et le tube externe (128) ;
un ballonnet (130) couplé au tube externe (128) ; et
une pointe (136) ;
**caractérisé en ce que**
l'ensemble à bobine (145) s'étend distalement au-delà de l'extrémité du ballonnet (130) et distalement le long d'une partie de la pointe (136).

2. Cathéter à ballonnet (100) selon la revendication 1, dans lequel la bobine (142) comprend une bobine de ruban.

3. Cathéter à ballonnet (100) selon la revendication 1 ou 2, dans lequel le revêtement (143) est disposé le long d'une surface intérieure de la bobine (142).

4. Cathéter à ballonnet (100) selon la revendication 3, dans lequel le revêtement (143) est disposé le long d'une surface extérieure de la bobine (142).

5. Cathéter à ballonnet (100) selon l'une quelconque des revendications 1 à 4, dans lequel le revêtement (143) inclut un polymère fluoré.

6. Cathéter à ballonnet (100) selon l'une quelconque des revendications 1 à 5, dans lequel la bobine (142) inclut de l'acier inoxydable.

7. Cathéter à ballonnet (100) selon l'une quelconque des revendications 1 à 6, dans lequel le revêtement (143) inclut du polytétrafluoroéthylène.

8. Cathéter à ballonnet (100) selon l'une quelconque des revendications 1 à 7, dans lequel la gaine (144) inclut un polyamide.

9. Cathéter à ballonnet (100) selon l'une quelconque des revendications 1 à 8, dans lequel la gaine (144) inclut un tube thermorétractable.

10. Cathéter à ballonnet (100) selon l'une quelconque des revendications 1 à 9, dans lequel l'ensemble à bobine (145) s'étend distalement jusqu'à une position adjacente à l'extrémité de la pointe (136), ou jusqu'à l'extrémité de la pointe (136).

11. Dispositif médical incluant un cathéter à ballonnet (100) selon l'une quelconque des revendications précédentes.
